# EUROPEAN PATENT APPLICATION

(11) **EP 4 850 715 A1**
(43) Date of publication of application: **09.12.2026**
(21) Application number: 26181649.0
(22) Date of filing: 28.05.2026
(51) Int. Cl.: A61M 1/16

(54) **RECYCLING OF OXYGEN FOR ECMO**

(30) Priority: 06.06.2025 US 202563818811 P
(71) Applicant: Breethe, Inc., Halethorpe, MD 21227 (US)
(72) Inventor: COURNANE, Stephen, Halethorpe, Maryland, 21227 (US); HARTRANFT, Evan, Halethorpe, Maryland, 21227 (US); ORIZONDO, Ryan, Danvers, 01923 (US); ZHANG, Jiafeng, Halethorpe, Maryland, 21227 (US)
(74) Representative: Wittmer, Maximilian

(57) **Abstract**

An extracorporeal membrane oxygenation (ECMO) system for infusing blood with oxygen and for removing carbon dioxide from blood includes a blood oxygenator. The blood oxygenator includes a supplemental system for collecting exhaust gas from oxygenator, removing carbon dioxide from the exhaust gas, and recycling excess oxygen back through the oxygenator.

## Description

### CROSS-REFERENCE TO RELATED APPLICATIONS

This application claims the benefit of the filing date of United States Provisional Patent Application No. 63/818,811 filed June 6, 2025, the disclosure of which is hereby incorporated herein by reference.

### BACKGROUND

The present disclosure relates to the recovery of a patient undergoing extracorporeal membrane oxygenation (ECMO), sometimes also referred to as extracorporeal life support (ECLS), and more particularly to an oxygenator used to oxygenate a patient's blood during ECMO therapy.

Patients awaiting or recovering from a heart or lung transplant and patients having conditions putting them at risk for heart or lung failure may be candidates for ECMO therapy. ECMO therapy is designed to address an excess of carbon dioxide and/or lack of oxygen in the blood where the lungs are not healthy and/or the heart cannot pump enough blood around the body. ECMO therapy is thus an extracorporeal technique which replaces these respiratory and cardiac functions in various situations ranging from support needed while separately treating the underlying causes of cardiac arrest to late-stage treatment for heart or lung failure. It may be a therapeutic treatment to provide temporary help when needed.

ECMO therapy operates by drawing deoxygenated blood from the body of a patient in a controlled manner to permit oxygenation of the blood and to remove carbon dioxide from the blood. The oxygenated blood is then cycled back into the patient. Generally, ECMO systems include a pump, an oxygenator, an oxygen source and a control center to monitor and control the process. It may also include a blood warmer to bring the blood back up to the temperature of the body as it is circulated back into the body. Deoxygenated blood drawn from the body via a cannula is pumped through an oxygenator where oxygen is infused in the blood and carbon dioxide is removed. The oxygenated blood is pumped from the oxygenator and back into the body, with blood warming in most instances.

### BRIEF SUMMARY

In accordance with an embodiment of a first aspect of the present disclosure, a blood oxygenation system includes a blood oxygenator, an oxygen source to provide oxygen through an inlet of the oxygenator, and a supplemental system configured to collect a first gas mixture from an outlet of the oxygenator, and the supplemental system forms a second mixture from the first gas mixture and provides the second gas mixture through the inlet of the oxygenator.

In another embodiment of the first aspect, the first gas mixture includes oxygen and carbon dioxide. In yet another embodiment of the first aspect, the second gas mixture includes at least 85% oxygen by concentration. In yet another embodiment of the first aspect, the second gas mixture includes oxygen, nitrogen, and argon. In yet another embodiment of the first aspect, the second gas mixture includes trace amounts of carbon dioxide.

In yet another embodiment of the first aspect, the supplemental system includes a filter to form the second gas mixture from the first gas mixture. The filter may remove carbon dioxide from the first gas mixture. The filter may be a metal-organic framework. In yet another embodiment of the first aspect, the supplemental system includes a collection device connected to the oxygenator for capturing the first gas mixture from the outlet of the oxygenator.

In yet another embodiment of the first aspect, the supplemental system includes a tank for storing at least a portion of second gas mixture. The supplemental system may include a pump for filing the tank with at least a portion of the second gas mixture. The supplemental system may include a valve for selectively providing the portion of the second gas mixture from the tank to the inlet of the oxygenator.

In accordance with an embodiment of a second aspect of the present disclosure, a blood oxygenation system includes an oxygenator having a housing with an interior, an oxygenator fiber bundle in the interior, a blood inlet to the bundle, a blood outlet, an oxygen inlet to the bundle for oxygenating the blood of the patient and a gas outlet; an oxygen source to provide oxygen to the oxygen inlet; a collection device to capture a gas discharged from the oxygenator at the oxygen outlet; and a gas recirculation system to provide a filtered oxygen from the captured gas to the oxygen inlet.

In another embodiment of the second aspect, the blood oxygenation system further comprises a pump for pumping oxygen through the oxygen inlet. In yet another embodiment of the second aspect, the bundle comprises an inner bundle of fibers and an outer bundle of fibers. The oxygen source may provide oxygen to the inner bundle of fibers, and the gas recirculation system may provide filtered oxygen to the outer bundle of fibers. The oxygen source may provide oxygen to the outer bundle of fibers, and the gas recirculation system may provide filtered oxygen to the inner bundle of fibers.

In yet another embodiment of the second aspect, the gas discharged from the oxygenator includes oxygen and carbon dioxide. In yet another embodiment of the second aspect, the filtered oxygen includes at least 85% oxygen by concentration. In yet another embodiment of the second aspect, the filtered oxygen partially includes nitrogen and argon. In yet another embodiment of the second aspect, the filtered oxygen includes trace amounts of carbon dioxide.

In yet another embodiment of the second aspect, the gas recirculation system includes a tank for storing at least a portion of filtered oxygen. The gas recirculation system may include a pump for filing the tank with at least a portion of the filtered oxygen. The gas recirculation system may include a valve for selectively providing the portion of the filtered oxygen from the tank to the oxygen inlet.

In accordance with an embodiment of a third aspect of the present disclosure, a blood oxygenator includes a housing having an oxygenator fiber bundle, a blood inlet to the bundle, a blood outlet, an oxygen inlet to the bundle for oxygenating the blood of a patient, and a gas outlet; and a supplemental device to collect a gas mixture from the gas outlet and recirculate at least a portion of oxygen from the gas mixture through the oxygen inlet.

In accordance with an embodiment of a fourth aspect of the present disclosure, a method of administering oxygen to blood of a patient includes directing the blood from a patient to oxygenator; supplying oxygen to the oxygenator from an oxygen supply to infuse the blood with oxygen; and recycling an exhaust gas from the oxygenator to provide supplemental oxygen to the oxygenator. In another embodiment of the fourth aspect, the recycling step includes removing carbon dioxide from the exhaust gas.

### BRIEF DESCRIPTION OF THE DRAWINGS

FIG. 1 is a schematic representation of an ECMO system in accordance with an embodiment of the present disclosure;
FIG. 2 is a perspective view of the ECMO system of FIG. 1;
FIG. 3 is a perspective view of a fiber bundle of an oxygenator in accordance with an embodiment of the present disclosure;
FIG. 4 is a cross-sectional schematic representation of the fiber bundle of FIG. 3;
FIG. 5 is a schematic representation of a recirculation system for an oxygenator in accordance with an embodiment of the present disclosure; and
FIG. 6 is a schematic representation of a recirculation system for an oxygenator in accordance with another embodiment of the present disclosure.

### DETAILED DESCRIPTION

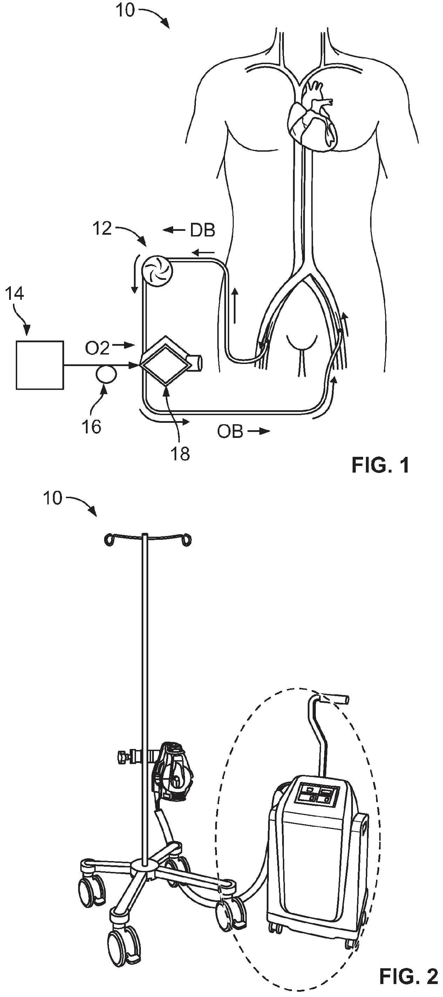

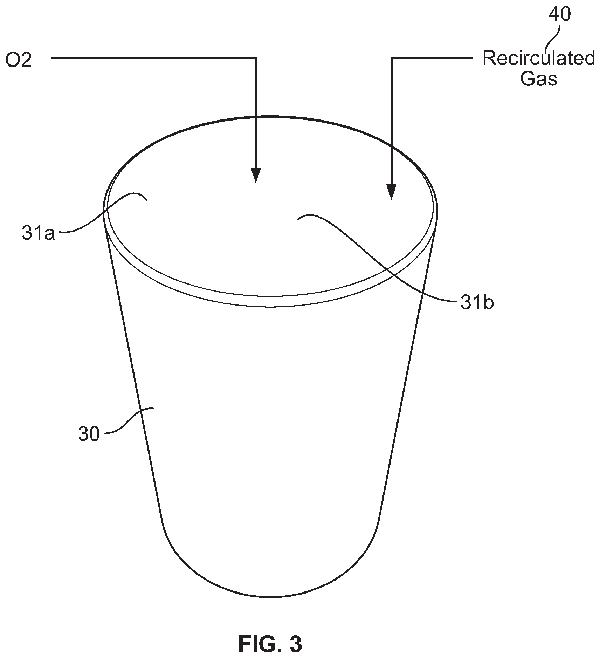

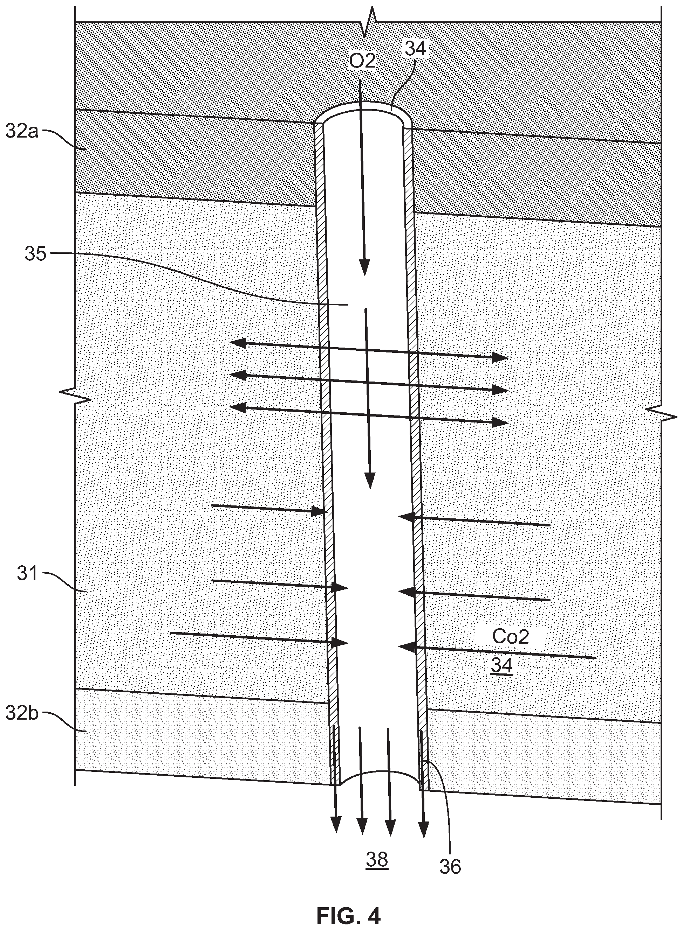

Oxygenators may be used to oxygenate a patient's blood during ECMO therapy. Oxygenators currently use bundles of semipermeable, hollow fibers that are designed for initiating a gas transfer of oxygen and carbon dioxide when contacting blood. For example, referring to FIGS. 1 and 2, there is illustrated an ECMO system generally designated as 10 which includes a blood pump 12, an oxygen source 14, an oxygen pump 16 and an oxygenator 18. Oxygenator 18 includes a hollow fiber bundle 31, as shown in FIGS. 3 and 4, made of semi-permeable hollow fibers, which is encased by an upper cap 32a and a lower cap 32b within the housing 30 to secure the hollow fibers in the housing 30. A gas conduit 35 extends between an oxygen inlet 34 and exhaust gas outlet 36. In use, as shown in FIGS. 1 and 4, deoxygenated blood DB is pumped to a blood inlet of the oxygenator 18, while oxygen is pumped from an oxygen source 14 to an oxygen inlet of the oxygenator 18. Oxygenated blood OB exits the oxygenator 18 at a blood outlet and is circulated back into the patient. Optionally, a blood warming device may be used to warm the blood being circulated back into the patient.

Also as shown in FIGS. 3 and 4, supply oxygen enters each respective fiber of fiber bundle 31 through an oxygen inlet 34 and is infused into the blood, while carbon dioxide is forced from the blood into the conduit 35 along with excess oxygen. In addition, the blood also imparts moisture and warmth into the conduit 35. An exhaust gas, designated as 38 in FIG. 4, is thus a mixture of warm and moist oxygen and carbon dioxide. Typically, such exhaust gas 38 is expelled into the environment through an exhaust gas outlet 36 of each respective fiber of fiber bundle 31. Additionally, the present disclosure contemplates any suitable arrangement of an oxygenator, such that any suitable fiber bundle may be used and/or the inlets and outlets can be oriented in any suitable arrangement.

The present disclosure is directed towards a method and system for recycling exhaust gas 38 from oxygenator 18 to supplement the oxygen received through the oxygen inlet 34. Typically, exhaust gas 38 can be more than 85% oxygen when oxygen source 14 is from a hospital wall supply or oxygen concentrator. However, this exhaust gas 38 will also typically include at least a 2% carbon dioxide concentration, and may also include concentrations of nitrogen and argon due to the supply oxygen not being pure. If exhaust gas 38 were recycled into oxygenator 18, concentrations of carbon dioxide would negatively impact the ability of fiber bundle 31 being able to remove carbon dioxide from the blood. Additionally, buildup of nitrogen and argon in the recycled gas may decreased the efficiency of oxygenator 18 due to a decrease in the oxygen concentration being supplied. Thus, if exhaust gas 38 is to be recycled through oxygenator 18, it is preferable to have a system and method for removing carbon dioxide from exhaust gas 38.

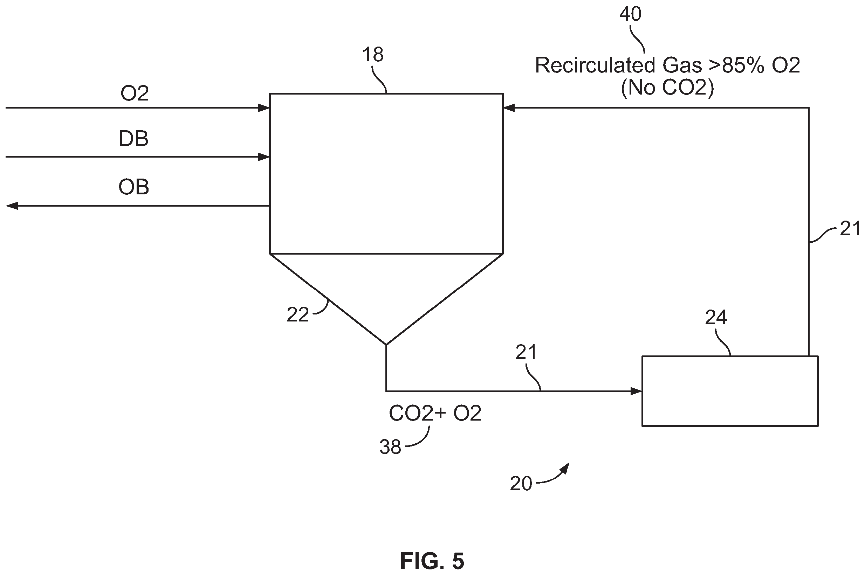

FIG. 5 illustrates schematically an embodiment of a supplemental or recirculation system 20 for recycling oxygen from an oxygenator 18 of ECMO system 10. Recirculation system 20 includes a gas collection device 22, a gas filter 24, and plurality of gas conduits 21 for transporting gas. In the depicted embodiment, a conduit 21 connects collection device 22 to filter 24, and another conduit 21 connects filter 24 to an inlet 34 of oxygenator 18.

Collection device 22 captures exhaust gas 38 from oxygenator 18. Thus, a collection device 22 can be positioned at exhaust gas outlet 36, either adjacently or in contact with it. Collection device 22 may be in the shape of a funnel. Additionally, collection device 22 may include a moisture trap and/or sponge for collecting excess moisture in the exhaust gas 38. For example, the collection device 22 may be a collection device or collection structure like that disclosed in U.S. Provisional Pat. Appl. No. 63/691,376, the disclosure of which is hereby incorporated by reference herein.

Filter 24 receives exhaust gas 38 from collection device 22 though conduit 21. As mentioned above, when exhaust gas 38 passes through filter 24, exhaust gas 38 primarily includes oxygen, but may also include carbon dioxide, nitrogen and/or argon. Filter 24 separates and removes carbon dioxide from exhaust gas 38 to form a recirculated gas 40 to be received through an inlet of oxygenator 18. Recirculated gas 40 is at least 85% oxygen and includes trace amounts of carbon dioxide.

Filter 24 may comprise a metal-organic framework (MOF) to effectively store and remove carbon dioxide from the exhaust gas 38. MOFs are used to remove carbon dioxide from the exhaust gas produced from fossil fuels due to their extremely high porosities and internal surface areas. Thus, the ultrahigh porosity of crystalline materials making up an MOF has been proven effective for storing and removing a relatively large amount of carbon dioxide. For example, the MOFs may be like those disclosed in U.S. Patent Nos. 11,731,077, the disclosure of which is hereby incorporated by reference herein. An example MOF that filter 24 may comprise is MOF-74. MOF-74 has a high surface area, typically around 1100 to 1500m2/g, which provides a large number of adsorption sites for gas molecules. The pore diameter of MOF-74 is approximately 8 to 11°A. This size is suitable for selectively adsorbing CO2, which can fit into the pores, while allowing O2 to pass through more freely. MOF-74 exhibits a higher affinity for CO2 compared to O2, making it effective for the separation of these gases. This is primarily due to the polarizability of CO2 and its ability to interact with the framework. Additionally, another example MOF that filter may comprise is a zeolitic imidazolate frameworks (ZIFs), such as ZIF-8. ZIF-8 is composed of magnesium, nickel and cobalt, and its chemical formula can be represented as M(C8H4O6)·*n*H2O.

Filter 24 may comprise, in place of MOF or in addition to MOF, other known devices or techniques for separating carbon dioxide from exhaust gas 38. Examples of the types of techniques that may be used by filter 24 include cryogenic separation, chemical reactions or absorption, and membrane separation. Cryogenic separation is a technique that exploits the different boiling points of carbon dioxide and oxygen by cooling the exhaust gas 38 to a very low temperature. This causes the carbon dioxide to condense into a liquid or solid, which can then be separated from the exhaust gas 38. Chemical reactions or absorption is a technique in which carbon dioxide is selectively interacted with certain chemical substances to form a new compound, which effectively removes the carbon dioxide from exhaust gas 38. For example, carbon dioxide reacts with calcium hydroxide to form calcium carbonate, and solvents such as an amines solution could be used to capture carbon dioxide from exhaust gas 38. Membrane separation is a technique which employs semi-permeable membranes that allow carbon dioxide to pass through more readily than oxygen. Thus, as the exhaust gas 38 is passed over the semi-membrane, carbon dioxide and oxygen are separated from one another.

As mentioned above, similar to the supply oxygen, recirculated gas 40 enters each respective fiber of fiber bundle 31 through an oxygen inlet 34. In one embodiment, as shown in FIG. 3, recirculated gas 40 may supply an outer bundle of fibers 31a of fiber bundle 31, while oxygen source 14 or an oxygen concentrator supplies an inner bundle of fibers 31b of fiber bundle 31. In other embodiments, recirculated gas 40 may supply inner bundle 31b, while oxygen source 14 supplies outer bundle 31a, or recirculated gas 40 and oxygen source concurrently supply the entire inner and outer bundles of fiber bundle 31.

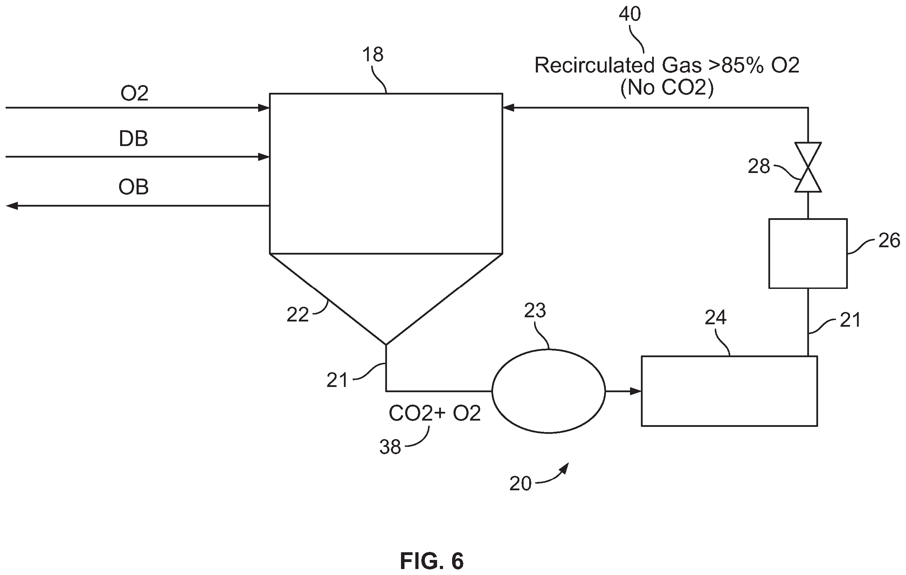

FIG. 6 illustrates schematically another embodiment of recirculation system 20. In addition to the gas collection device 22, gas filter 24, and plurality of gas conduits 21 for transporting gas, the embodiment of recirculation system 20, as shown in FIG. 6, also includes a storage pump 23, a storage tank 26, and a gas valve 28. Pump 23 allows for oxygen or recirculated gas 40 to be pressurized and stored in tank 26 within recirculation system 20. A control system for ECMO system 10 may determine when higher flows of oxygen are required for oxygenator 18, such that valve 28 is opened to release the stored recirculated gas 40 and increase flow rate into oxygenator 18. Thus, a control system automatically controls valve 28 to maintain a pressure in tank 26 and provide supplemental recirculated gas 40 to oxygenator 18 as adjustably desirable. Additionally, a control system may regularly purge the recirculated gas 40 stored in tank 26 to prevent a buildup of nitrogen and argon in recirculated gas 40. Such recirculated gas 40 may be purged to the atmosphere or elsewhere.

The present disclosure contemplates any suitable positioning and use of various optional components, such as filters, pumps, and valves. Additionally, the present disclosure contemplates the use of any suitable warming system, humidifying system, and sterility system. For example, humidity systems may be used to control or monitor the humidity of recirculated gas 40 within recirculation system 20 with moisture traps, desiccants or other chemical absorbents, vapor filters, and supplemental semi-permeable membranes. In other examples, sterilization systems may be used to sterilize and prevent the growth of microbes within recirculated gas 40 with the use of heating elements, high efficiency particulate air (HEPA) filters, and irradiative methods using ultraviolet (UV) light or any other suitable form of electromagnetic radiation.

Although the present invention has been described with reference to particular embodiments, these embodiments are merely illustrative. There are many embodiments that can be developed and are encouraged to be developed yet remain within the spirit and scope of the claims which follow.

## Claims

1. A blood oxygenation system, comprising:
a blood oxygenator;
an oxygen source to provide oxygen through an inlet of the oxygenator; and
a supplemental system to collect a first gas mixture from an outlet of the oxygenator, and
the supplemental system forms a second mixture from the first gas mixture and provides the second gas mixture through the inlet of the oxygenator.

2. The blood oxygenation system of claim 1, wherein
the first gas mixture includes oxygen and carbon dioxide.

3. The blood oxygenation system of claims 1 or 2, wherein
the second gas mixture includes at least 85% oxygen by concentration.

4. The blood oxygenation system of any one of claims 1 to 3, wherein
the second gas mixture includes oxygen, nitrogen, and argon.

5. The blood oxygenation system of any one of claims 1 to 4, wherein
the second gas mixture includes trace amounts of carbon dioxide.

6. The blood oxygenation system of any one of claims 1 to 5, wherein
the supplemental system includes a filter to form the second gas mixture from the first gas mixture.

7. The blood oxygenation system of claim 6, wherein
the filter removes carbon dioxide from the first gas mixture.

8. The blood oxygenation system of claim 6 or 7, wherein
the filter is a metal-organic framework.

9. The blood oxygenation system of any one of claims 1 to 8, wherein
the supplemental system includes a collection device connected to the oxygenator for capturing the first gas mixture from the outlet of the oxygenator.

10. The blood oxygenation system of any one of claims 1 to 9, wherein
the supplemental system includes a tank for storing at least a portion of second gas mixture.

11. The blood oxygenation system of claim 10, wherein
the supplemental system includes a pump for filing the tank with at least a portion of the second gas mixture.

12. The blood oxygenation system of claims 10 or 11, wherein
the supplemental system includes a valve for selectively providing the portion of the second gas mixture from the tank to the inlet of the oxygenator.

13. The blood oxygenation system of any one of claims 1 to 12, comprising:
wherein the oxygenator has a housing with an interior, an oxygenator fiber bundle in the interior, a blood inlet to the bundle, a blood outlet, an oxygen inlet to the bundle for oxygenating the blood of the patient and a gas outlet;
an oxygen source to provide oxygen to the oxygen inlet;
a collection device to capture a gas discharged from the oxygenator at the oxygen outlet; and
a gas recirculation system to provide a filtered oxygen from the captured gas to the oxygen inlet.

14. The blood oxygenation system of claim 13, wherein
the bundle comprises an inner bundle of fibers and an outer bundle of fibers,
the oxygen source provides oxygen to the inner bundle of fibers, and
the gas recirculation system provides filtered oxygen to the outer bundle of fibers.

15. The blood oxygenation system of claim 13, wherein
the bundle comprises an inner bundle of fibers and an outer bundle of fibers,
the oxygen source provides oxygen to the outer bundle of fibers, and
the gas recirculation system provides filtered oxygen to the inner bundle of fibers.
